# EUROPEAN PATENT SPECIFICATION

(11) **EP 2 229 589 B1**
(45) Date of publication and mention of the grant of the patent: **10.06.2015**
(21) Application number: 08860005.1
(22) Date of filing: 10.12.2008
(51) Int. Cl.: G01N 33/48, G01N 33/53

(54) **DISEASE PATHWAY-BASED METHOD TO GENERATE BIOMARKER PANELS TAILORED TO SPECIFIC THERAPEUTICS FOR INDIVIDUALIZED TREATMENTS**
AUF KRANKEITSSTOFFWECHSELWEGEN BASIERENDE VERFAHREN ZUR ERZEUGUNG VON AUF SPEZIFISCHE THERAPEUTIKA ZUGESCHNITTENEN BIOMARKERGRUPPEN ZUR INDIVIDUALISIERTEN BEHANDLUNG
PROCÉDÉ BASÉ SUR UN MÉCANISME DE MALADIE POUR GÉNÉRER DES GROUPES DE MARQUEURS BIOLOGIQUES CONÇUS POUR DES THÉRAPIES SPÉCIFIQUES

(30) Priority: 12.12.2007 US 13249 P; 09.12.2008 US 331356
(43) Date of publication of application: 22.09.2010
(73) Proprietor: Blume-Jensen, Peter, Westfield, NJ 07090 (US)
(72) Inventor: Blume-Jensen, Peter, Westfield, NJ 07090 (US)
(74) Representative: Winter, Brandl, Fürniss, Hübner, Röss, Kaiser, Polte - Partnerschaft mbB
(86) International application number: PCT/US2008/086283
(87) International publication number: WO 2009/076472

(56) References cited:
- WO-A2-2007/015947
- GARMAN KATHERINE S ET AL: "Genomic strategies for personalized cancer therapy.", 15 October 2007 (2007-10-15), HUMAN MOLECULAR GENETICS 15 OCT 2007 LNKD- PUBMED:17911165, VOL. 16 SPEC NO. 2, PAGE(S) R226 - R232, XP002664141, ISSN: 0964-6906 * abstract; figures 1-4 *
- WEST MIKE ET AL: "Embracing the complexity of genomic data for personalized medicine", GENOME RESEARCH, COLD SPRING HARBOR LABORATORY PRESS, WOODBURY, NY, US, vol. 16, no. 5, 1 May 2006 (2006-05-01), pages 559-566, XP002538622, ISSN: 1088-9051, DOI: 10.1101/GR.3851306
- VAN 'T VEER LAURA J ET AL: "Enabling personalized cancer medicine through analysis of gene-expression patterns", April 2008 (2008-04), NATURE (LONDON), VOL. 452, NR. 7187, PAGE(S) 564-570, XP002664142, ISSN: 0028-0836 * abstract; figures 1,2 *
- DUFFY MICHAEL J ET AL: "A Personalized Approach to Cancer Treatment: How Biomarkers Can Help", November 2008 (2008-11), CLINICAL CHEMISTRY, VOL. 54, NR. 11, PAGE(S) 1770-1779, XP002664143, ISSN: 0009-9147
- RECKAMP ET AL.: 'Tumor response to combination celecoxib and erlotinib therapy in non-small cell lung cancer is associated with a low baseline matrix metalloproteinase-9 and a decline in serum-soluble E-cadherin.' J THORAC ONCOL. vol. 3, no. 2, February 2008, pages 117 - 124, XP009136632
- KRYSAN ET AL.: 'Prostaglandin E2 activates mitogen-activated protein kinase/Erk pathway signaling and cell proliferation in non-small cell lung cancer cells in an epidermal growth factor receptor-independent manner.' CANCER RESEARCH vol. 65, no. 14, 15 July 2005, pages 6275 - 6281, XP008137633

## Description

### PRIORITY CLAIM

This application claims priority to U.S. Patent Application No. 12/331,356, filed December 9, 2008, by Peter Blume-Jensen, entitled "DISEASE PATHWAY-BASED METHOD TO GENERATE BIOMARKER PANELS TAILORED TO SPECIFIC THERAPEUTICS FOR INDIVIDUALIZED TREATMENTS", and to U.S. Provisional Patent Application No. 61/013,249, filed December 12, 2007, by Peter Blume-Jensen, entitled "DISEASE PATHWAY-BASED METHOD TO GENERATE BIOMARKER PANELS TAILORED TO SPECIFIC THERAPEUTICS FOR INDIVIDUALIZED TREATMENTS".

### FIELD OF THE INVENTION

The present invention relates to methods of treating diseases based on identifying and establishing disease pathway-oriented biomarkers and biomarker tools that semi-quantitatively measure the effect and intersection point of specific therapeutics on disease pathways and that are also predictive for efficacy in treating specific patient populations with a particular exogenous substance, including, but not limited to biologics, biologics-derived, and synthetic therapeutics.

### BACKGROUND OF THE INVENTION

During the last decade an increasing number of so-called 'Targeted Therapeutics' have been developed. These are treatments directed to correct or abrogate the underlying molecular defects driving specific diseases while causing only minimal unwanted effects. However, most diseases are molecularly heterogeneous, and so only a fraction of patients with a certain disease share an underlying molecular disease mechanism. As a consequence, pharmaceutical and biotech companies are now facing an enormous challenge: how to identify the specific sub-group of patients with a certain disease that are likely to respond to their specific targeted therapeutics.

In the field of oncology, more than 220 targeted therapeutics are currently in clinical development, and it is predicted that less than 9% of these will make it to the market. This is primarily due to the inability to predict efficacy and identify responders. Hence, the biggest attrition occurs in phase IIB, which is the stage of clinical trials where efficacy is assessed. It takes on average 7 years to bring a new project through successful phase IIB, and a failed phase IIB oncology drug costs on average $M 150-280. Also in other therapeutic areas the attrition rate for therapeutics is highest in Phase IIB clinical trials.

### BRIEF DESCRIPTION OF THE FIGURES

The details of one or more embodiments are set forth in the description below. Other features, objects and advantages will be apparent from the description, the drawings, and the claims.
**Figure 1** is a schematic showing the proposed approach according to an embodiment of the invention;
**Figure 2** is a schematic showing a classical classification of cancers based on the anatomical tissue of origin of specific cancer types;
**Figure 3** is a schematic showing a new classification of cancers based on the various pathway alterations that are involved in specific cancer types based on an embodiment of the invention;
**Figure 4** shows a representative flow scheme of the processes used to identify and generate pathway-based biomarkers for a specific cancer therapeutic;
**Figure 5** illustrates an example of a therapeutic that inhibits two different signaling pathways and consequent alterations in signaling pathway activity distally of the drug interception level as measured using phospho-specific antibodies;
**Figure 6** shows an example of two dynamic phosphoantibody biomarkers measuring the drug target exposure, and two exclusion phosphoantibodies that indicate undesirable pathway activity;
**Figure 7** illustrates an example of a therapeutic that inhibits two different signaling pathways and consequent alterations in signaling pathway activity distally of the drug interception level as measured using phospho-proteomics or other pathway activation-state read-out;
**Figure 8** illustrates how the drug-induced pathway alterations provide basis for generating dynamic phosphoantibody biomarkers measuring the drug target exposure, as well as exclusion phosphoantibodies that indicate undesirable pathway activity;
**Figure 9** illustrates a real example of a malignant melanoma patient harboring different oncogenic mutations and who is being treated with a MEK inhibitor;
**Figure 10** illustrates the use of a dynamic phosphoantibody biomarker and exclusion phosphoantibodies for a malignant melanoma patient treated with a MEK inhibitor;
**Figure 11** illustrates a cell line which harbors a deregulated pathway causing a disease. Upon drug inhibition or genetic downregulation, an alternative pathway leading to the disease is activated;
**Figure 12** illustrates the cell line shown in **Figure 11** which harbors a deregulated pathway causing a disease. Despite drug inhibition or genetic downregulation, the same conserved disease-causing 'by-pass' mechanism can be activated as that shown in **Figure 11****;**
**Figure 13** illustrates the cell line shown in **Figure 12** which harbors the deregulated disease-causing pathway and how drug inhibition or genetic downregulation of targets block the main and alternative pathways leading to the disease; and
**Figure 14** shows a representative flow scheme of the processes used to identify and generate pathway-based biomarkers for a specific therapeutic.

### DETAILED DESCRIPTION OF THE INVENTION

Prior to setting forth the invention, it may be helpful to an understanding thereof to first set forth definitions of certain terms that are used hereinafter.

"Biomarker" means a molecule that indicates activity of a disease pathway. It is most typically, but not necessarily, translated from RNA. Increased abundance or specific post-translational modification of the Biomarker indicates that the disease signaling pathway activity has been up regulated. Decreased abundance or specific post-translational modification of the Biomarker indicates that the disease signaling pathway activity has been down regulated.

"Biomarker Responder Package" means a panel of biomarker predictive of response to a therapeutic and also dynamically regulated in response to the therapeutic. The package enables responder stratification in trials and target exposure monitoring of the therapeutic

"Cancer Space" means most pathways which result in the cancer.

"Combinatorial Targeted Therapeutics" means a combination of targeted therapeutics that when used together have additive or synergistic treatment effect

"Disease" means a pathological condition of a mammal which leads to a debilitating condition of the mammal caused by a perturbation of a genetic pathway.

"Disease Space" means most pathways which result in the disease.

"Phosphoantibodies" are antibodies that are directed against and specifically recognize phosphorylation of a specific amino acid(s) in a specific amino acid sequence of a specific protein. Phosphorylation is one of several post-translational modifications and indicates the activity state in a disease pathway of a particular protein.

"Activation-state antibodies" are antibodies that are directed against and specifically recognize modifications of cellular molecules, most often proteins, that alter the activity-state of said molecule. These modifications are typically post-translational modifications and indicate the activity state in a disease pathway of a particular protein.

"Phosphoproteomics" is a mass spectrometry-based method to identify and semi-quantitatively or qualitatively measure the phosphorylation state of individual proteins within a pool of proteins.

"Phosphosignatures" are specific peptides residing within proteins in a cell, wherein one or more of the peptide residues is phosphorylated. The pattern of phosphorylated peptides in a cell constitutes a characteristic phosphosignature.

"Post-Translational Product" means the product of a RNA translation process that has been subsequently modified in a post-translational event

"Translational Product" means the product of a RNA translation process.

"Responder Identification" means the ability to identify the patients that will be effectively treated with a particular drug or exogenous agent. This can be achieved by use of the 'biomarker responder package'.

"Specific Disease" means a pathological condition caused by on one or several perturbed signaling pathways.

"Therapeutic" or "drug" means an exogenous agent intended to be administered to a diseased mammal. A drug that interferes with the signaling activity of a disease pathway involved in a specific disease, allows matching of the right drug with the right patients, based on a profiling of the drug's activity on said disease signaling pathways.

The inability to identify responder patients for targeted therapeutics that are active in smaller and smaller disease markets together with increasing costs for bringing drugs to market, has led to decreasing return of investment for Pharmaceutical companies. On top of that, patent expirations leading to generics competition and problems for Health Insurance companies to predict and reimburse patient treatment expenditures, has led to the realization of the urgent need for a method to stratify and segregate patients to ensure efficacy of therapeutics. In fact, there is a push from Health Insurance companies to have efficient methods to identify the patients that will respond to a therapeutic, and there are emerging examples of reimbursement being contingent on efficacy of a particular therapeutic. Finally, there is a huge emotional and societal impact with the present methodology where patients are being treated with in-efficient drugs.

Due to the challenges in making 'block buster 'drugs, there is a need and desire for new and preferably improved therapeutics for defined patient populations. The key challenge for the successful market launch of these new targeted therapeutics is to be able to identify the responders, both during clinical trials and for drugs on the market.

In order to address the development of a therapeutic for a specific patient population, an approach is required that enables optimal selection of responder patients in much smaller phase I clinical trials. These trials can be designed to also assess response to the drug. This will enable much quicker go, no-go decisions, and ultimately result in more efficient therapeutic development thus reducing therapeutic development costs.

A related, but unique problem is that of identifying the optimal combination of targeted therapeutics that will provide maximal treatment efficacy for specific diseases. Most diseases are not possible to completely cure by interfering with just one molecular mechanism or target, but often several targets need to be modulated by several therapeutics to provide optimal treatment efficacy and prevent so-called drug resistance or 'by-pass' to kick in.

Previously, a genetics approach using genomics technologies has been used to correlate specific genetic alterations with specific disease phenotypes. While extremely powerful and able to identify most changes at the genetic level with specific diseases, these changes are associative and not necessarily causally involved in the disease phenotype. Hence, it is usually an enormous challenge to identify the best molecular drug targets through a genetic analysis. Consequently, relatively few successful predictive genetic biomarker approaches have been identified.

In an embodiment of the invention, the activity of specific intracellular signal transduction pathways can be linked with the observed disease phenotype. Since these pathways are the effectors of cellular behavior they are causally involved in the specific disease phenotype. In various embodiments of the invention, a pathway-based approach can be used for Identification of the appropriate Responder to a drug. In various embodiments of the invention, a pathway-based approach can be used for identification of optimal Combinatorial Targeted Therapeutics and the associated biomarker responder package. In an embodiment of the invention, a pathway-based approach can be used for rational selection of patients going into clinical trials. In an alternative embodiment of the invention, a pathway-based approach can be used for selecting patients that will be most effectively treated by one or more drugs. The pathway-based approach will allow stratification of patients and selection of only those patients responding to the right combinations of treatments.

### Definition of a Specific Disease

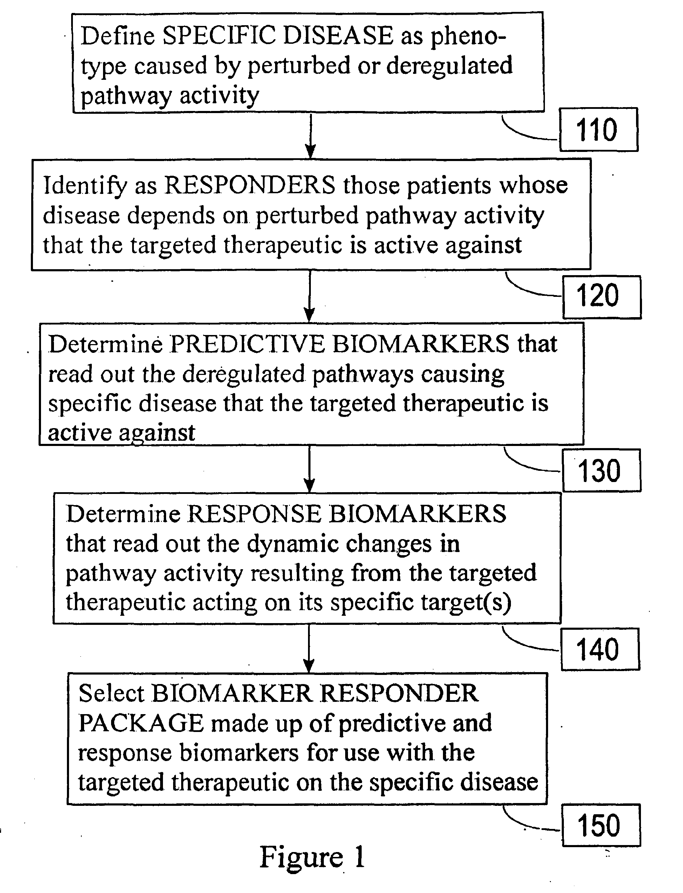

Rather than defining a specific disease based on the tissue of origin and its histopathological appearance, the specific disease **110** is defined based on the perturbed signaling pathways that cause the specific disease phenotype as shown in **Figure 1****.** Based on this pathway definition of disease, a specific disease can be classified into several sub-groups or fractions, each having a specific pattern of perturbed or deregulated pathway activity. For instance, specific types of cancer, like breast, colorectal or prostate, each can be stratified into several sub-groups that are characterized by a certain signaling pathway activity pattern. Likewise, specific types of inflammatory, auto-immune, neurological, and other diseases can be sub-grouped based on a shared deregulated or perturbed signaling pathway pattern within a specific sub-group of patients with a specific disease. Since the perturbed pathway activity pattern is causing the disease phenotype, it is possible to link the effect of a particular drug on the activity of specific signaling pathways with its ability to cause a desirable therapeutic effect. If the drug modulates the perturbed pathway activity linked to a sub-fraction of patients **120** with a specific disease **110,** that particular drug is expected to be effective in treating that sub-fraction of patients with the particular disease. Conversely, if the drug does not modulate the signaling activity of (an)other pathway(s) causally involved in the disease phenotype it is not expected to be effective for treating said patient sub-population.

### Predictive and Dynamic Biomarkers

Based on this concept, the signaling pathways that a particular drug interferes with, can be identified and the predictive biomarkers **130** and dynamic biomarkers **140** that can read the activity of these pathways before and after drug treatment can be derived (see **Figure 1****).** Likewise, certain core pathways that the drug does not interfere with and that are known to be causally involved in a particular disease(s) can be identified, and biomarkers derived for those pathways in order to be able to exclude these patients that suffer from a disease (in which those drug non effected pathways are involved) from being treated with the specific drug in question.

### Biomarker Responder Package

Thus for a specific drug, a Biomarker Responder Package **150** is made up of a collection or panel of predictive biomarkers **130** and dynamic biomarkers **140** that can be derived for use with a specific drug to act on the specific disease where the Biomarker Responder Package can read the activity of these pathways before and after drug treatment (see **Figure 1**). In various embodiments of the invention, the predictive or dynamic biomarkers can be antibodies. In an embodiment of the invention, the predictive or dynamic biomarkers can be antibodies directed against phosphorylated or otherwise post-translationally modified proteins. For a specific drug, a simple constellation of phosphorylation state-specific or other activation state-specific antibodies can be derived and used to identify the key nodes of signaling activity that are compatible with beneficial therapeutic efficacy and also those that can preclude efficacy of the drug. In an embodiment of the invention, approximately 3 to approximately 20 activation-state antibodies can predict the response of a mammal to a therapeutic. In an embodiment of the invention, 4 to 8 activation-state antibodies can predict the response of a mammal to a therapeutic.

In alternative embodiments of the invention, a biomarker package can contain other tools in addition to, or instead of activation-state antibodies that are able to identify and directly or indirectly measure the level of activity of specific signaling pathways. Examples include, but are not limited to phosphoproteomics and other mass spec-based approaches, reporter assays based on chemiluminescence, fluorescence, radioactivity, and other reporter signal, degradation of signaling proteins by ubiquitination, and other proteasome-mediated processes, scaffold and chaperone protein cargo proteins.

### Method I. Identification, generation, and application of predictive and dynamic biomarkers for a specific therapeutic.

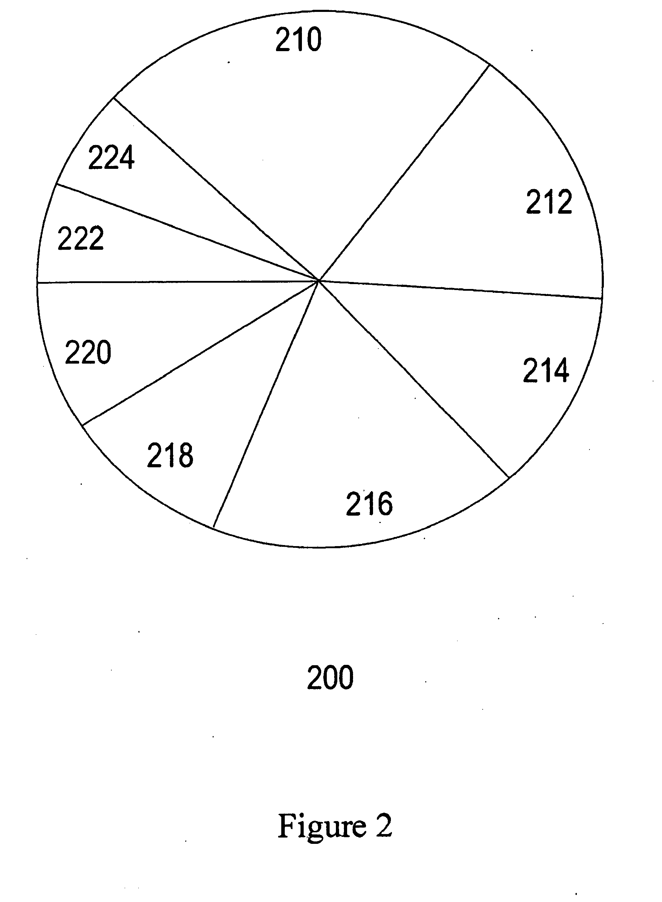

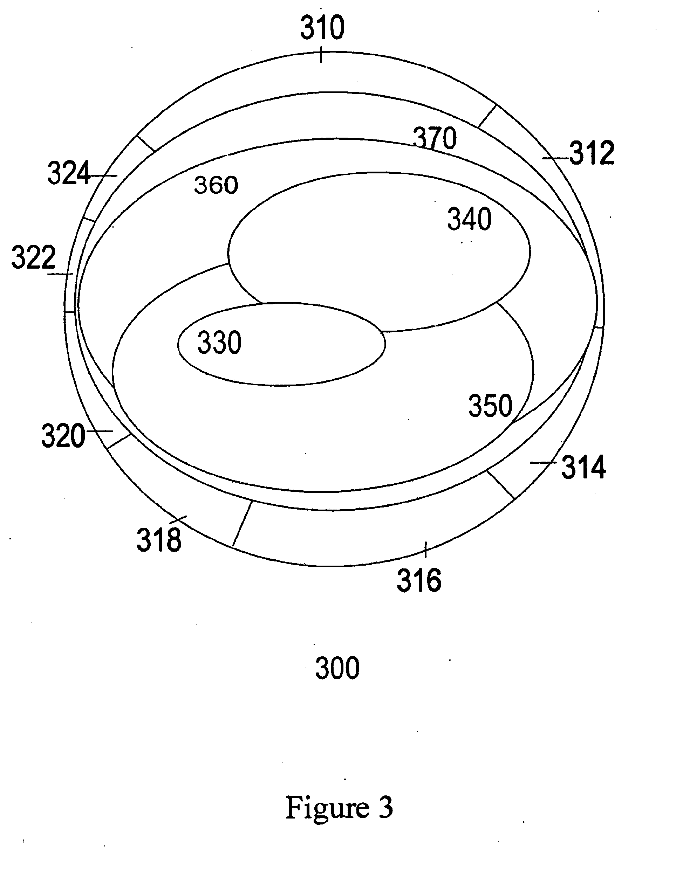

This method rests on the ability to redefine and represent various diseases, including cancer, inflammatory disorders, autoimmune diseases, neurological disorders as diseases of perturbed pathway activity. Various molecular genetic lesions or variations characteristic for specific diseases are the cause of specific pathway alterations, and these, in turn, are the mediators of the disease phenotype. As shown in **Figure 2****,** cancers **200** can be classified based on the specific cancer type as prostrate **210,** lung **212,** breast **214,** colorectal **216,** endometrial **218,** sarcomas **220,** leukemia **222,** and other solid **224.** Alternatively, as shown in **Figure 3****,** this classification of cancers (as prostrate **310,** lung **312,** breast **314,** colorectal **316,** endometrial **318,** sarcomas **320,** leukemia **322,** and other solid **324)** can be overlaid with a new classification based on the various pathway alterations that are involved in a pathway including JAK-STAT **330,** Src **340,** IKK-NFkB **350,** Ras-Raf-ERK **360,** Core PI3'K **370.** Likewise, inflammatory, autoimmune, and neurological disorders can be classified based on specific pathway alterations and perturbations.

Most available information about the involvement of certain pathway alterations in specific diseases stem from laboratory and clinical molecular and genetic studies, supplemented by a growing amount of information from genetic and proteomic systematic studies and databases. Based on this information the key pathway alterations involved in major diseases have been identified, and can be modeled in engineered or naturally occurring cells and cell lines. This collection of engineered and natural cells and cell lines are generated to cover most known pathway alterations involved in specific diseases, and they are at the core of the approach.

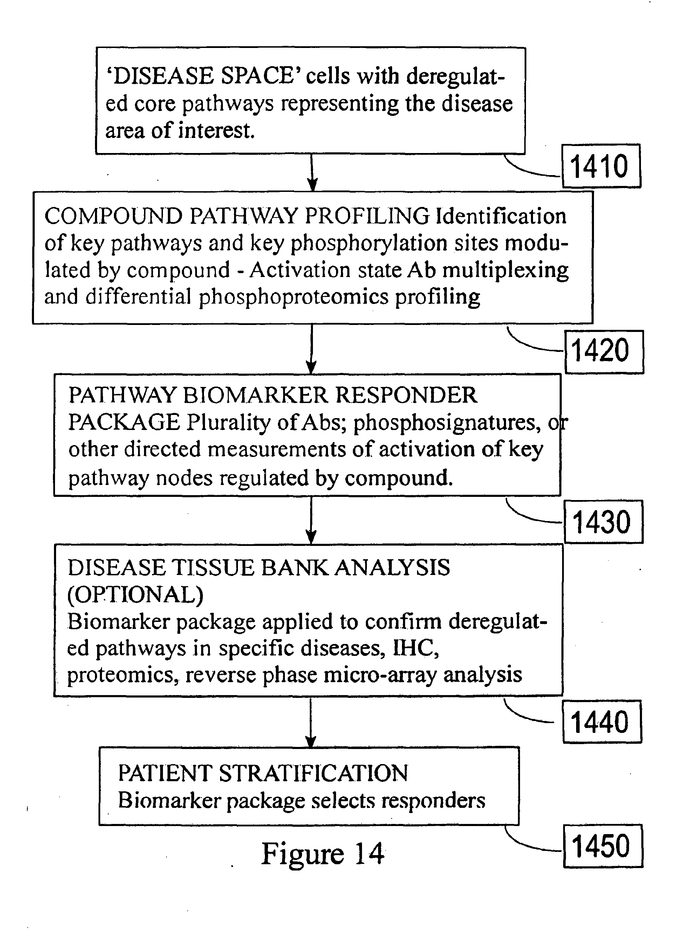

**Figure 14** shows an embodiment of the invention, where a representative flow scheme can be used to identify and generate pathway-based biomarkers for a specific therapeutic against a disease, where the disease is first identified **1410,** and next compound action against disease pathways is profiled **1420,** next the biomarker responder package is selected **1430,** and the patient stratification involving steps **1410-1450.** Step **1440** is optional where the biomarker responder package **1430** is applied in tissue to confirm the deregulated or non functional pathway in a mammal with the disease.

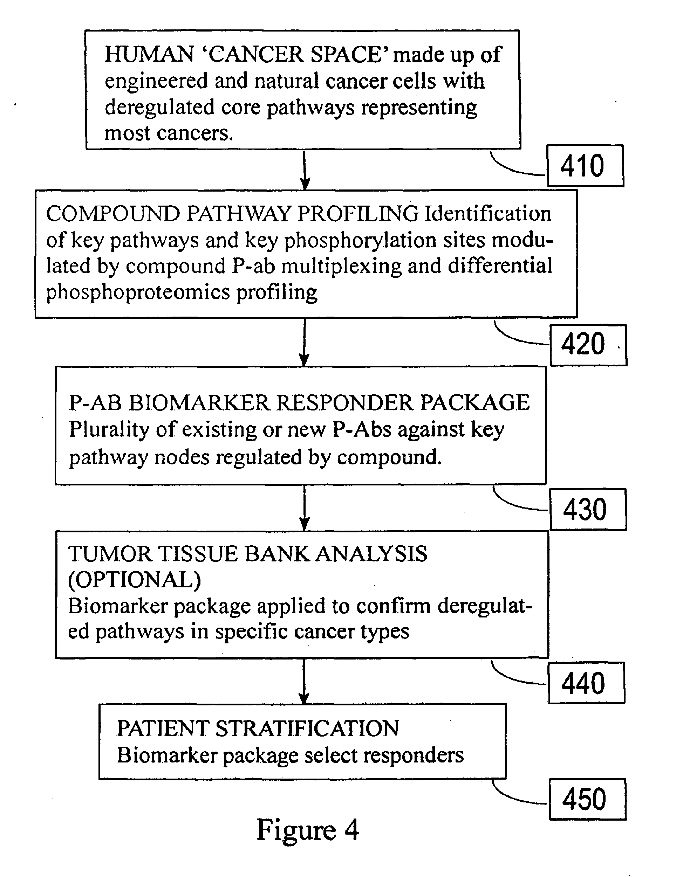

**Figure 4** shows an embodiment of the invention, where a representative flow scheme can be used to identify and generate pathway-based biomarkers for a specific therapeutic, where the cancer space is first identified **410,** and next compound action against disease pathways is profiled **420,** next the phosphoantibody responder package is selected **430,** and the patient stratification involving steps **410-450.** Step **440** is optional where the antibody responder package **430** is applied in tissue to confirm the deregulated or non functional pathway in the specific form of cancer.

### I. Disease Space Coverage

In the first step, cell lines are obtained or generated from cells that have specific core pathway alterations relevant for a certain disease. For instance, deregulated core phosphoinositide 3' kinase (PI3'K) signaling is causally involved in a major fraction of solid and hematopoietic cancers. A number of genetic gain-of-function (GOF) and loss-of-function (LOF) mutations in human cancer cause deregulated core PI3'K signaling. These include, but are not limited to LOF of the tumor suppressor PTEN, GOF mutations of PI3'K, either through mutations in the regulatory or catalytic subunits of PI3'K, amplifications and GOF mutations of the serine/threonine protein kinase PKB, also called Akt, amplifications of the serine/threonine protein kinase p70S6K, LOF of the tumor suppressor protein TSC1/2. Accordingly, human cells and cell lines are engineered to harbor these GOF and LOF mutations through (inducible) cDNA over expression (GOF mutations) or (inducible) knock down (LOF) through usage of inducible, lentiviral shRNA directed against the specific mRNA. As a control, the same cell line that these mutations are introduced into, can be kept unmodified, as a matched pair control. In addition, a number of human cancer cell lines have been identified and isolated from human cancer patients with deregulated PI3'K signaling, so these naturally-occurring cancer cell lines can be part of the cellular repertoire to cover relevant PI3'K pathway alterations. By extension of this approach major cancer core pathways known to be relevant for specific cancers, including, but not limited to canonical Ras-Raf-MAPK signaling (a number of solid and hematopoietic cancers have deregulated Ras signaling), deregulated JAK-STAT signaling (numerous hematopoietic malignancies and myeloproliferative disorders), deregulated Src kinase signaling (hematopoietic malignancies), deregulated IKK-NFkB signaling (multiple myeloma, plasma cell disorders, other hematopoietic malignancies, liver carcinoma) can be addressed. In addition, relevant mutations in cell surface proteins and receptors, in particular in receptor protein tyrosine kinases, will be modeled in the cell lines. Most pathways relevant for cancer in mammals, so-called 'cancer space' **(****Figures 2** and **3****)** can be determined. By linking specific pathway alterations with specific mammalian forms of cancer, and have these pathway alterations modeled into cell lines and cells, most forms of cancer can be represented.

The same approach is used to generate cell lines and cells with deregulated pathway alterations relevant for other diseases, and hence representing the disease space for the particular type of disease under investigation, e.g. inflammatory, autoimmune, neurological. Finally, the custom-engineered and natural cells and cell lines representing the disease space are carefully characterized to ensure that they have the expected and proper pathway deregulation. This is primarily done by phosphopathway analysis using commercially available phosphor-antibodies (P-Abs). A vast number of P-Abs directed against specific phosphoproteins involved in deregulated core pathways have been generated over the years, and they cover the major signaling pathway nodes. In various embodiments of the invention, each patient population suffering from a disease where a cell line collection can be used to identify pathways of action of an exogenous agent can be determined.

### II. Compound pathways profiling

The cellular modeling of disease space by deregulated pathways will enable the identification and measurement of the effects of a particular therapeutic agent on specific pathways. This can be done through P-Ab multiplexing with P-Abs, phosphoproteomics analysis to identify phosphosignatures, and other probes for measuring pathway activity. This information, in turn, is useful for a number of purposes including:

a. confirmation of the suspected on-target(s) for the therapeutic by the expected pathway effects;

b. identification of potentially unknown 'off-target' activity by effects on pathways that are not related to the known 'on-target(s)'. This information can be crucial in identifying potentially new therapeutic area opportunities, through the connection between specific pathways and specific diseases, based on the above pathway representation and definition of disease;

c. identification of dynamically regulated phosphosignatures or other post-translational pathway modifications. These, in turn, are the basis for generation of dynamic pathway biomarkers, e.g. phosphoantibodies, directed phosphoproteomics measurements, and other directed pathway 'probes';

d. identification of pathways that are not affected by the therapeutic. Through the causal association of these pathways with specific diseases, this enables the generation of so-called exclusion pathway biomarkers. These are pathway probes, e.g. phosphoantibodies or other activation-state antibodies, directed phosphoproteomics, or other measurements of the pathway activity, that the therapeutic agent is inactive against. To the extent that these pathways are involved in the disease phenotype, exclusion biomarkers can be applied to exclude patients with this pathway activity from the specific treatment, since the agent is inactive against these.

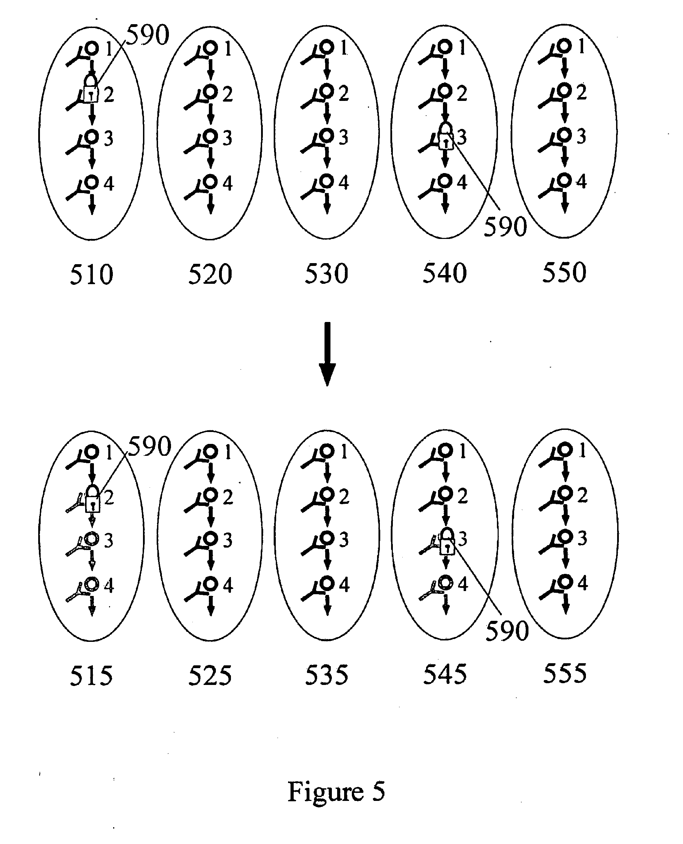

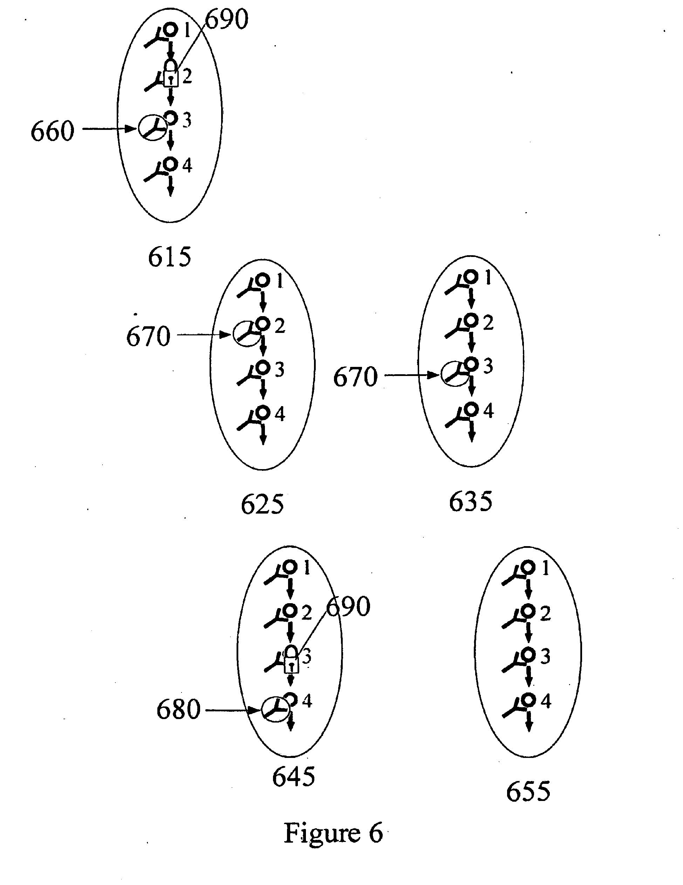

An example of compound profiling in a Disease Space, where the P-TEN-PI3'K **510, 610, 710, 810** Ras-Raf-ERK **520, 620, 720, 820** IKK-NFkB **530, 630, 730, 830** JAK-STAT **540, 640, 740, 840** and Src **550, 650, 750, 850** pathways are shown in **Figures 5-8****. A** compound **590, 690, 790, 890** that is known to inhibit a PI3'K pathway **515, 615, 715, 815** target is used as an example. As illustrated in **Figure 6****,** the compound **690** is confirmed to hit the PI3'K pathway **615,** resulting in decreased phosphorylation distal in the PI3'K pathway, as measured with P-Abs **660.** The compound does not effect the Ras-Raf-ERK **525, 625, 725, 825** IKK-NFkB **535, 635, 735, 835** and Src **555**, **655, 755, 855** pathways. However, the compound is shown to also interfere with core JAK-STAT pathway signaling **545, 645, 745, 845** as measured with P-Ab **680.** In an embodiment of the invention, this profiling can identify a potential new disease indication for the compound, namely diseases where perturbed JAK-STAT signaling **645** is involved. The dynamic and exclusion biomarkers can be derived from dynamic P-Abs **660** and/or exclusion phosphoantibodies **670.**

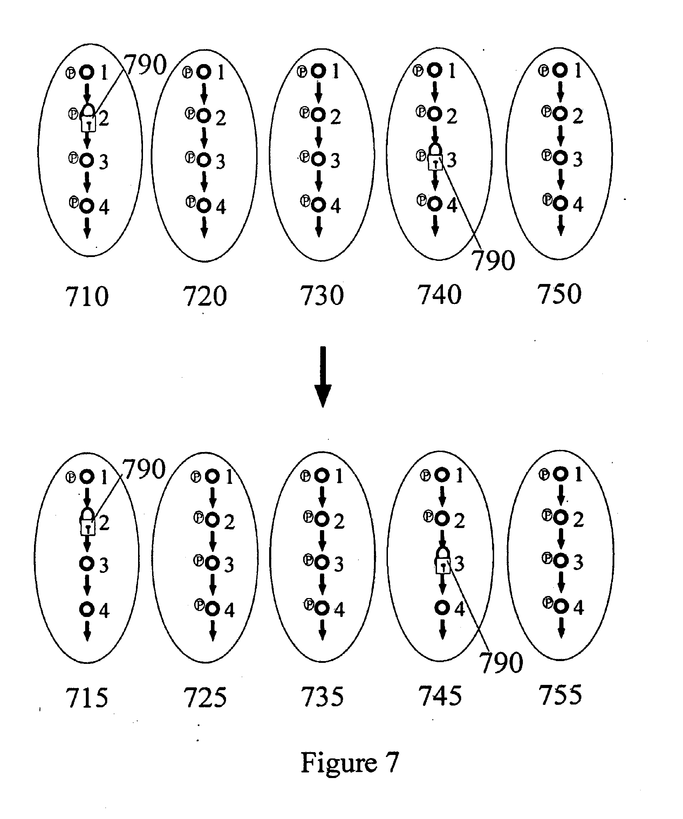

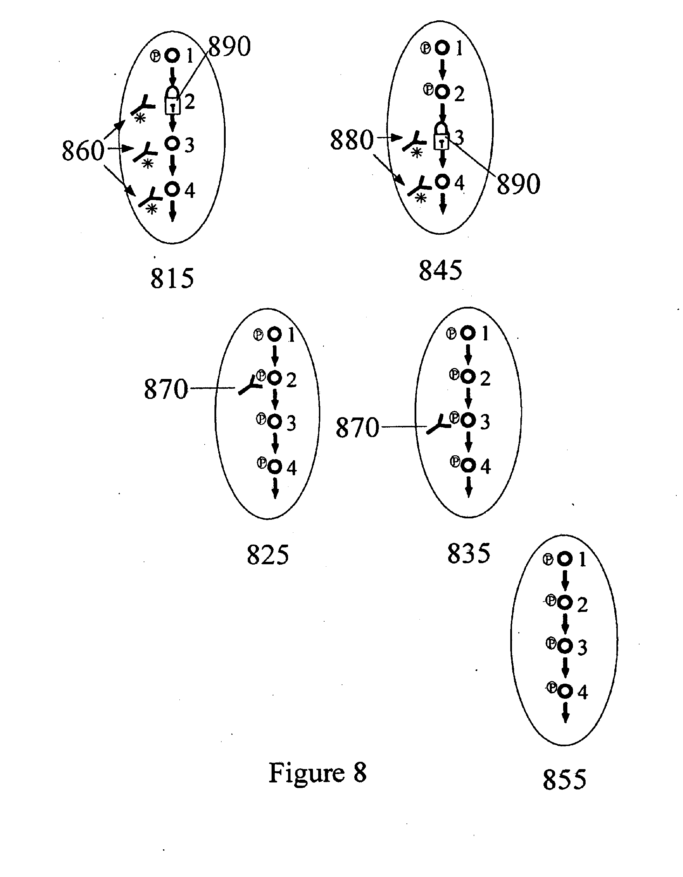

In an alternative embodiment of the invention, illustrated in **Figures 7** and **8****,** the compound **790, 890** is confirmed to hit the PI3'K pathway **715, 815** resulting in decreased phosphorylation distal in the PI3'K pathway, as measured with directed differential phosphoproteomics **860.** However, the compound is also shown to interfere with core JAK-STAT pathway signaling **745, 845** as measured with directed differential phosphoproteomics **880.** In an alternative embodiment of the invention, this profiling can identify a potential new disease indication for the compound, namely diseases where perturbed JAK-STAT signaling **745, 845** is involved. The dynamic and exclusion biomarkers can be derived from dynamic phosphosignatures **860, 880** and exclusion phosphosignatures **870.**

### III. Pathway Biomarker Responder Package

The panel of dynamic and exclusion biomarkers together constitutes a 'biomarker package' that when used together on diseased tissue will enable a rational prediction of therapeutic efficacy by the specific therapeutic agent that was profiled **(****Figures 6** and **8****).** In essence, this is a simple, custom-generated predictive and response biomarker package, consisting of a panel of biomarkers tailored for the therapeutic agent. The package will be validated by application to the cellular model of disease space to confirm the expected pathway alterations. Once validated, this biomarker package can be applied on disease tissues and on biopsies from patients or sick animals entering clinical trials to ensure segregation of responders from non-responders, as described in IV and V below.

### IV. Disease Tissue Bank Analysis

The pathway biomarker responder package can be applied to relevant human or animal disease tissue banks. Typically these are paraffin embedded, more rarely cryo-preserved after OCT mounting. The purpose of this is to confirm that the perturbed pathway activity pattern that is specifically measured with the biomarker package is recognized in the relevant disease tissue. In particular one or two of the biomarkers in the biomarker package, which can consist of 4-8 predictive biomarkers, might not confirm that the particular pathway activity is perturbed as in the cellular model of the disease space. This information can be used to go back and repeat steps I to III above to identify additional biomarkers to replace the non-confirmatory biomarkers. While there can be many reasons for such a lack of confirmation, the most likely is that the cells are grown artifactually in two dimensions on a plastic dish, and hence many signaling pathways are not regulated as in adherent cells growing as part of the disease tissue. This caveat is difficult to overcome, but one way to partially overcome this is through analysis of a number of cell lines and cells where the same pathway perturbation is achieved through different genetic alterations relevant for the disease of interest.

### V. Patient Stratification

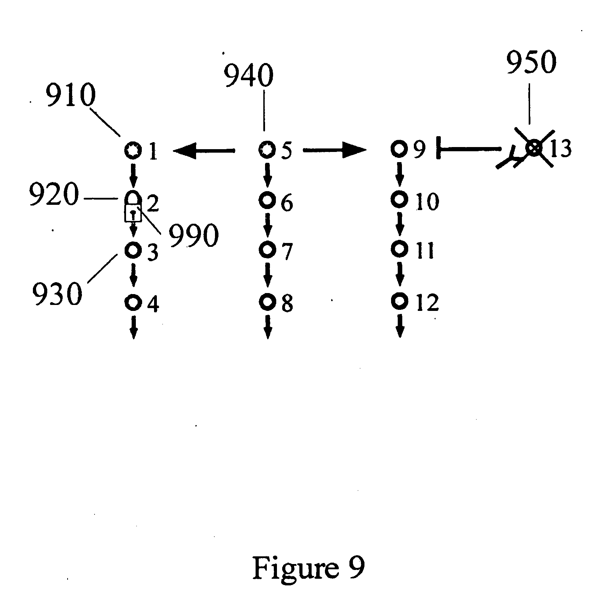

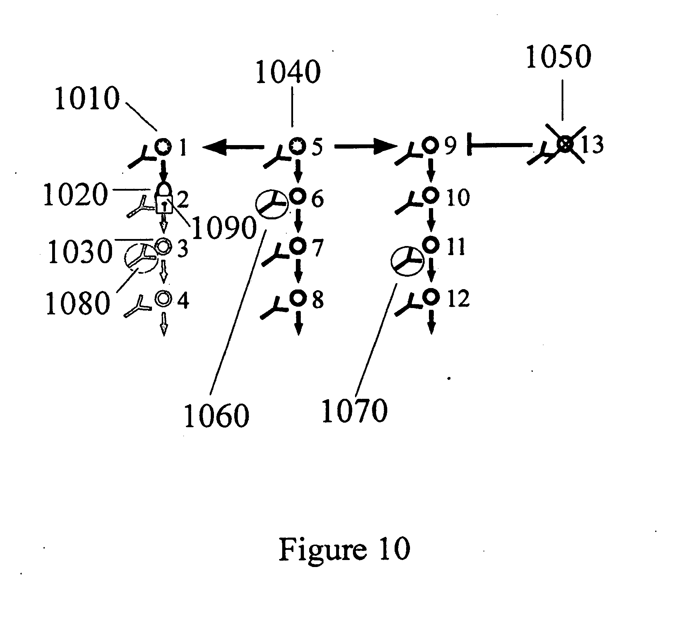

The ultimate goal of the generated biomarker responder package is to be able to apply it to patient tissue to select the patients that will respond to the specific therapeutic agent. The biomarker package is purposely as simple as possible with the highest predictive power such that it can be used to stratify patients in early clinical trials and also be marketed hand-in-hand with the specific therapeutic agent. In clinical trials, the biomarker package will ideally be applied on diseased tissue before and after treatment with the therapeutic agent it was developed for, so that Bayesian principles can be applied to further improve its predictive power even from a very small, yet stratified patient material. As an example, see **Figures 9** and **10****.** Malignant melanoma is a cancer originating in pigment cells, so-called melanocytes, of the skin. Over 66% of malignant melanoma patients have been found to harbor a GOF B-Raf (V600E) mutation **910, 1010** rendering the serine/threonine kinase B-Raf constitutively active. This particular mutation will result in deregulated signaling through MEK **920, 1020** and ERK **930, 1030** and so in principle patients with GOF mutation of B-Raf should be responsive to a MEK inhibitor **990, 1090,** and the dynamic biomarker applied to monitor MEK inhibitor target exposure is a phoshoantibody directed against the substrate of MEK, called ERK **1080.** Accordingly, in clinical trials where malignant melanoma patients have a GOF-B-Raf mutation as the sole mutation, a number of patients exhibit disease stabilization and even partial regression upon MEK inhibitor treatment. However, a number of patients with GOF B-Raf mutations have concurrent GOF Ras mutations **940** and/or concurrent LOF PTEN mutations **950.** These mutations result in deregulated PI3'K signaling, as measured with the P-Ab against target 11, and deregulated GTPase signaling, as measured with the P-Ab against target 6 **1060.** Since these pathways are themselves involved in malignant transformation of cells and cancer, it is important to exclude patients with these pathways active, since the MEK inhibitor does not act on these. Accordingly, an example of how a derived P-Ab biomarker package can be used to identify responder patients for a specific MEK inhibitor for malignant melanoma, is illustrated in Table I. In an embodiment of the invention, in pre-treatment biopsies patients with high signals from exclusion P-Abs against targets 6 and 11 **1060** and **1070** are excluded from treatment, while patients with high signals from P-Ab against P-ERK **1080** are included for treatment. In an alternative embodiment of the invention, in addition to the predictive and dynamic biomarkers, one or more additional biomarker can be used involving target-directed PCR against the main known mutated target genes, namely b*-raf, ras,* and *p-ten* to confirm that the biomarker package is applied to a disease with the relevant key mutations involved in the perturbed pathway alterations.

**Table I. Stratification Principle based on antibody assay for determining Responder Patient Population**

| Antibody # (from **Figure 10**) | Label in **Figure 10** | Pre Treatment Assay | Post Treatment Assay | Exclude Patients |
|---|---|---|---|---|
| 3 | 1080 | +++ | (+) | none |
| 6 | 1060 | (+) | (+) | ++ or +++ |
| 11 | 1070 | (+) | (+) | ++ or +++ |

### Method II

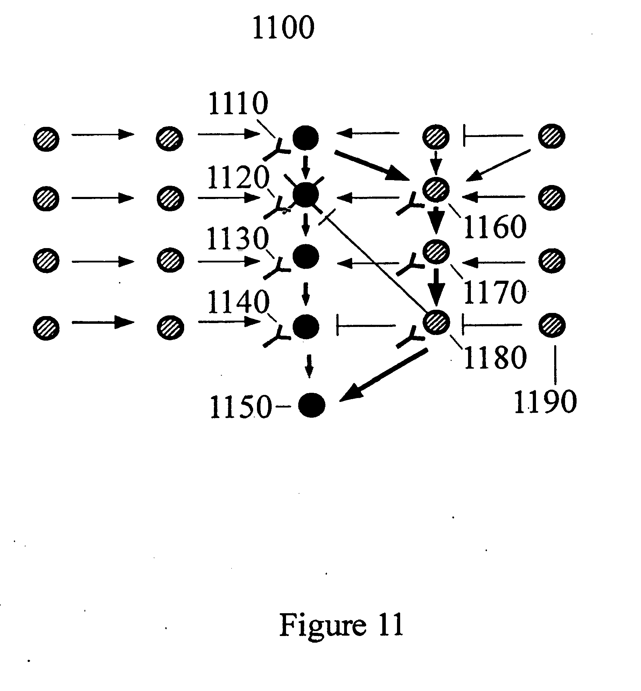

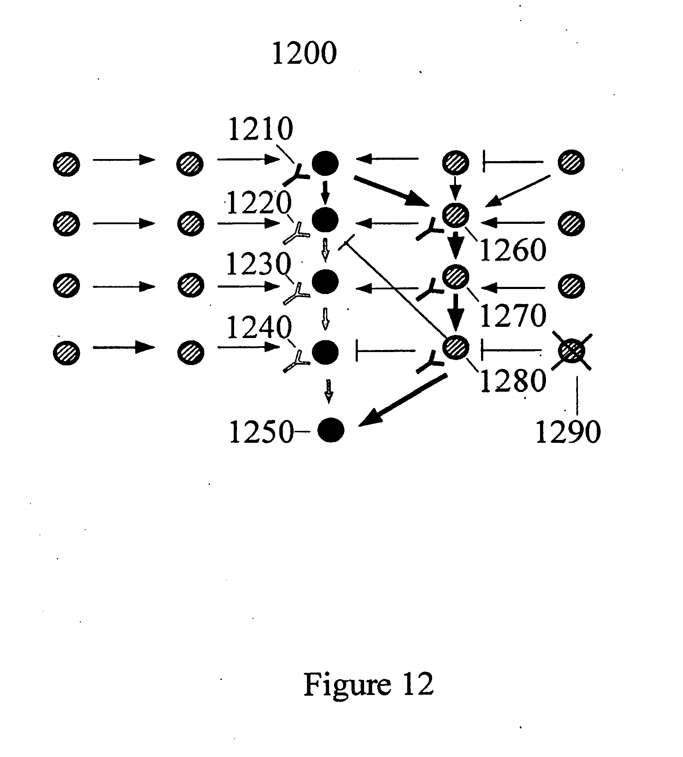

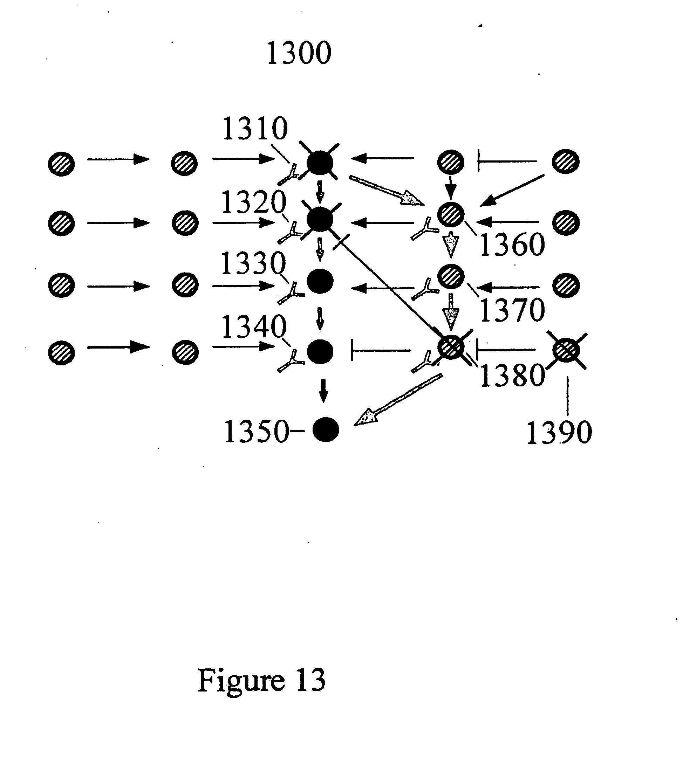

Identification of optimal target combinations with associated predictive biomarkers for diseases. The cancer space coverage by the collection of pathway context cell lines and cells can also be used to identify optimal target combinations to inhibit or modulate simultaneously to prevent by-pass pathway activity and to derive the optimal biomarker package for agents hitting such target combination. The starting point is based on defining a particular pathway of interest based on its involvement in and relevance for a particular disease(s). For instance, if the disease of interest is cancer, optimal target combinations for the various mutations that result in deregulated core PI3'K and Ras-Raf-MAPK signaling could be the focus. A number of the cell lines in the disease space collection will harbor these deregulated pathways **1100, 1200, 1300,** see **Figures 11-13****.** Cell lines containing a particular pathway perturbation of interest are interrogated because of their relevance for a disease of interest. Through multiparameter P-Ab analysis, differential global phosphoprofiling, or other pathway read-outs, the overall pathway activity inside the cells of interest are monitored. To assess whether a particular target, e.g. target 2 in this example, is a potential attractive target for inhibition of deregulated pathway activity, that particular target is inactivated through (inducible) knock down. The LOF in essence mimicks a therapeutic agent targeting that protein. As shown in **Figure 11****,** as a consequence of the target inhibition of inhibited pathway **1110-1150** a 'rescue' or 'by-pass' pathway **1160-1180** is immediately activated, as measured with the pathway monitoring approach. Similarly, as shown in **Figure 12****,** inhibition of a target not involved in the pathway **1190** does not alter the status quo and either the initial pathway **1210-1250** or a rescue pathway **1260-1280** can be activated. This pathway activation is undesirable if it can potentially be involved in a disease phenotype. In an embodiment of the invention, in order to prevent this by-pass mechanism from kicking in, systematic testing of each of the core pathway targets through (inducible) knock down individually, followed by multiparameter pathway analysis can be carried out. An optimal combination of the directed target knock downs can be achieved (see **Figure 13**) when they result in quenching of major pathway activity **1310-1380** irrespective of whether they also knock down other targets **1390.** Based on the same principle as used above to derive biomarkers for specific therapeutic agents, the optimal biomarker package to be used for therapeutic combinations that would hit the optimal combination of targets can be derived. Based on the identification of the optimal target combinations through mimicking of a therapeutic through genetic knock down, this method is particularly attractive for RNAi therapeutics. Assuming that the delivery issue for RNA-based therapeutics will soon be solved, this would be an ideal method for identifying optimal target combinations for RNA-based therapeutic cocktails for specific diseases, and generate the optimal associated biomarker package for these.

### Method III

Pathway interceptor screening approach for identification of clinically relevant new targets for specific diseases. In another embodiment of the present invention, new targets relevant for a specific disease can be identified using bioinformatics-derived target libraries optimized for the likelihood of targets that interact with the core deregulated pathway causing a disease of interest. Through inducible, lentiviral shRNA knock down of all targets in the library the effects of this downregulation is measured through phosphopathway analysis by phosphoantibody multiplexing of the core deregulated pathways. Targets that when inducibly knocked down cause decreased signaling through the core deregulated disease pathway modeled in the cell line(s) of study, will be candidates for clinically relevant therapeutics development for said disease. As the screening approach is outlined in PCT Application WO/2005/103299 "RNAi-Based Target Identification and Validation", inventor: Blume-Jensen, P.) which is expressly incorporated by reference in its entirety.

In an embodiment of the invention, a method of identifying a responder patient population for treatment with an exogenous agent comprises: establishing a cellular model of disease space based on one of more signaling pathway, identifying the effect of the exogenous agent in the one or more signaling pathway, determining a biomarker responder package including a plurality of biomarkers, wherein the plurality of biomarkers are specific for one or more of the signaling pathway, assaying the state of the one or more signaling pathway with the biomarker responder package before and after drug dosing and identifying the responder patient population that will be responsive for the exogenous agent based on the assay.

In an alternative embodiment of the invention, a method of identifying a responder patient population for treatment with an exogenous agent comprises: establishing a cellular model of disease space based on one of more signaling pathway, identifying the effect of the exogenous agent in the one or more signaling pathway, determining a biomarker responder package including a plurality of biomarkers, wherein the plurality of biomarkers are specific for one or more of the signaling pathway, wherein one or more of the signaling pathways is perturbed, assaying the state of the one or more signaling pathway with the biomarker responder package before and after drug dosing and identifying the responder patient population that will be responsive for the exogenous agent based on the assay.

In another embodiment of the invention, a method of identifying a responder patient population for treatment with an exogenous agent comprises: establishing a cellular model of disease space based on one of more signaling pathway, identifying the effect of the exogenous agent in the one or more signaling pathway, determining a biomarker responder package including a plurality of biomarkers, wherein the plurality of biomarkers are specific for one or more of the signaling pathway, wherein one or more of the signaling pathways is active, assaying the state of the one or more signaling pathway with the biomarker responder package before and after drug dosing and identifying the responder patient population that will be responsive for the exogenous agent based on the assay.

In a further embodiment of the invention, a method of identifying a responder patient population for treatment with an exogenous agent comprises: establishing a cellular model of disease space based on one of more signaling pathway, identifying the effect of the exogenous agent in the one or more signaling pathway, determining a biomarker responder package including a plurality of biomarkers, wherein the biomarker responder package contains between:
a lower limit of three and an upper limit of twenty biomarkers, wherein the plurality of biomarkers are specific for one or more of the signaling pathway, assaying the state of the one or more signaling pathway with the biomarker responder package before and after drug dosing and identifying the responder patient population that will be responsive for the exogenous agent based on the assay.

In an embodiment of the invention, a method of identifying a responder patient population for treatment with an exogenous agent comprises: establishing a cellular model of disease space based on one of more signaling pathway, identifying the effect of the exogenous agent in the one or more signaling pathway, determining a biomarker responder package including a plurality of biomarkers, wherein the biomarker responder package contains one or more exclusion biomarkers and one or more dynamic biomarkers, wherein the plurality of biomarkers are specific for one or more of the signaling pathway, assaying the state of the one or more signaling pathway with the biomarker responder package before and after drug dosing and identifying the responder patient population that will be responsive for the exogenous agent based on the assay.

In another embodiment of the invention, a method of identifying a responder patient population for treatment with an exogenous agent comprises: establishing a cellular model of disease space based on one of more signaling pathway, identifying the effect of the exogenous agent in the one or more signaling pathway, determining a biomarker responder package including a plurality of biomarkers, wherein the biomarker responder package contains one or more predictive biomarkers, wherein the plurality of biomarkers are specific for one or more of the signaling pathway, assaying the state of the one or more signaling pathway with the biomarker responder package before and after drug dosing and identifying the responder patient population that will be responsive for the exogenous agent based on the assay.

In a further embodiment of the invention, a method of identifying a responder patient population for treatment with an exogenous agent comprises: establishing a cellular model of disease space based on one of more signaling pathway, identifying the effect of the exogenous agent in the one or more signaling pathway, determining a biomarker responder package including a plurality of biomarkers, wherein the plurality of biomarkers are specific for one or more of the signaling pathway, assaying the state of the one or more signaling pathway with the biomarker responder package before and after drug dosing and identifying the responder patient population that will be responsive for the exogenous agent based on the assay, wherein the assay detects one or more signaling pathways that are down regulated by drug treatment.

In an alternative embodiment of the invention, a method of identifying a responder patient population for treatment with an exogenous agent comprises: establishing a cellular model of disease space based on one of more signaling pathway, identifying the effect of the exogenous agent in the one or more signaling pathway, determining a biomarker responder package including a plurality of biomarkers, wherein the plurality of biomarkers are specific for one or more of the signaling pathway, assaying the state of the one or more signaling pathway with the biomarker responder package before and after drug dosing and identifying the responder patient population that will be responsive for the exogenous agent based on the assay, wherein the assay detects one or more signaling pathways that are down regulated by drug treatment, wherein based on the assay, patients are excluded from the responder patient population based on the inability of the drug to modulate said disease pathways.

In an embodiment of the invention, a method of identifying a responder patient population for treatment with an exogenous agent comprises: establishing a cellular model of disease space based on one of more signaling pathway, identifying the effect of the exogenous agent in the one or more signaling pathway, determining a biomarker responder package including a plurality of biomarkers, wherein the plurality of biomarkers are specific for one or more of the signaling pathway, assaying the state of the one or more signaling pathway with the biomarker responder package before and after drug dosing and identifying the responder patient population that will be responsive for the exogenous agent based on the assay, wherein the assay detects one or more disease-relevant signaling pathways that are up regulated.

In various embodiments of the invention, a method of identifying a responder patient population for treatment with an exogenous agent comprises: establishing a cellular model of disease space based on one of more signaling pathway, identifying the effect of the exogenous agent in the one or more signaling pathway, determining a biomarker responder package including a plurality of biomarkers, wherein the plurality of biomarkers are specific for one or more of the signaling pathway, assaying the state of the one or more signaling pathway with the biomarker responder package before and after drug dosing and identifying the responder patient population that will be responsive for the exogenous agent based on the assay, wherein the assay detects one or more disease-relevant signaling pathways that are up regulated, wherein based on the assay patients are included in the responder patient population.

In an embodiment of the invention, a method of identifying a responder patient population for treatment with an exogenous agent comprises: establishing a cellular model of disease space based on one of more signaling pathway, identifying the effect of the exogenous agent in the one or more signaling pathway, determining a biomarker responder package including a plurality of biomarkers, wherein the plurality of biomarkers are specific for one or more of the signaling pathway, wherein the biomarkers are pathway activity state biomarkers, assaying the state of the one or more signaling pathway with the biomarker responder package before and after drug dosing and identifying the responder patient population that will be responsive for the exogenous agent based on the assay.

In another embodiment of the invention, a method of identifying a responder patient population for treatment with an exogenous agent comprises: establishing a cellular model of disease space based on one of more signaling pathway, identifying the effect of the exogenous agent in the one or more signaling pathway, determining a biomarker responder package including a plurality of biomarkers, wherein the plurality of biomarkers are specific for one or more of the signaling pathway, wherein the biomarkers are antibodies directed against post-translationally modified proteins, assaying the state of the one or more signaling pathway with the biomarker responder package before and after drug dosing and identifying the responder patient population that will be responsive for the exogenous agent based on the assay.

In a further embodiment of the invention, a method of identifying a responder patient population for treatment with an exogenous agent comprises: establishing a cellular model of disease space based on one of more signaling pathway, identifying the effect of the exogenous agent in the one or more signaling pathway, determining a biomarker responder package including a plurality of biomarkers, wherein the plurality of biomarkers are specific for one or more of the signaling pathway, wherein the biomarkers are antibodies directed against phospho proteins, assaying the state of the one or more signaling pathway with the biomarker responder package before and after drug dosing and identifying the responder patient population that will be responsive for the exogenous agent based on the assay.

## Claims

1. An in-vitro method of identifying a responder patient population for treatment with an exogenous agent comprising:
(a) establishing a cellular model of disease space based on one or more signaling pathway;
(b) identifying the effect of the exogenous agent in the one or more signaling pathway;
(c) determining a biomarker responder package including a plurality of biomarkers, wherein the plurality of biomarkers are specific for one or more of the signaling pathway, and the biomarkers are antibodies directed against post- translationally modified proteins;
(d) assaying the state of the one or more signaling pathway with the biomarker responder package before and after drug dosing; and
(e) identifying the responder patient population that will be responsive for the exogenous agent based on the assay.

2. The method of claim 1, wherein one or more of the signaling pathways is perturbed or active.

3. The method of claim 1, wherein the biomarker responder package contains between:
a lower limit of three; and an upper limit of twenty.

4. The method of claim 1, wherein the biomarker responder package contains one or more exclusion biomarkers for identifying a pathway that are not affected by the exogenous agent and one or more dynamic biomarkers for identifying dynamic changes in the pathway activity resulting from the exogenous agent.

5. The method of claim 1, wherein the biomarker responder package contains one or more predictive biomarkers for identifying a deregulated pathway.

6. The method of claim 1, wherein the assay detects one or more signaling pathways that are down regulated by drug treatment.

7. The method of claim 6, wherein based on the assay, patients are excluded from the responder patient population based on the inability of the drug to modulate said disease pathways.

8. The method of claim 1, wherein the assay detects one or more disease-relevant signaling pathways that are up regulated.

9. The method of claim 8, wherein based on the assay patients are included in the responder patient population.

10. The method of claim 1, wherein the biomarkers are pathway activity state biomarkers.

11. The method of claim 1, wherein the biomarkers are antibodies directed against phospho proteins.

12. An in-vitro method for identification of a mammal responsive to a drug for treating a disease comprising:
(a) establishing a cellular model of disease space for the disease based on one or more signaling pathway;
(b) determining a biomarker responder package including a plurality of biomarkers, wherein the biomarkers are specific for one or more of the signaling pathway and the biomarkers are antibodies directed against post-translationally modified proteins;
(c) taking a tissue obtained from the mammal;
(d) assaying the tissue with the biomarker responder package; and
(d) identifying if the mammal is responsive to the drug based on the assay.

13. An in-vitro method for identifying a mammalian population responsive to a drug for treating a disease comprising:
(a) establishing a cellular model of disease space for the disease based on one of more signaling pathway;
(b) determining a biomarker responder package including a plurality of biomarkers, wherein the biomarkers are specific for one or more of the signaling pathway and the biomarkers are antibodies directed against post-translationally modified proteins;
(c) identifying a cell line representative of the mammalian population;
(c) assaying the drug in vitro in the cell line with the biomarker responder package; and
(d) identifying if the mammalian population is responsive to the drug based on the assay.

## Patentansprüche

1. Ein in vitro Verfahren zum Identifizieren einer Responder-Patientenpopulation zur Behandlung mit einem exogenen Mittel, umfassend:
(a) Etablieren eines zellulären Modells eines Krankheitsraumes, basierend auf einem oder mehreren Signalwegen;
(b) Identifizierung der Wirkung des exogenen Mittels auf den einen oder mehreren Signalweg;
(c) Bestimmen eines Biomarker-Responder-Paketes einschließlich einer Mehrzahl von Biomarkern, wobei die Mehrzahl der Biomarker spezifisch für einen oder mehrere Signalwege sind und die Biomarker Antikörper sind, welche gegen posttranslational modifizierte Proteine gerichtet sind;
(d) Testen des Zustandes des einen oder der mehreren Signalwege mit dem Biomarker-Responder-Paket vor und nach Arzneimitteldosierung; und
(e) Identifizierung der Responder-Patientenpopulation, welche auf das exogene Mittel reagiert, basierend auf dem Testverfahren.

2. Verfahren nach Anspruch 1, wobei einer oder mehrere der Signalwege gestört oder aktiv ist.

3. Verfahren nach Anspruch 1, wobei das Biomarker-Responder-Paket zwischen einem unteren Grenzwert von drei und einem oberen Grenzwert von zwanzig enthält.

4. Verfahren nach Anspruch 1, wobei das Biomarker-Responder-Paket einen oder mehrere Ausschluss-Biomarker zum Identifizieren eines Weges enthält, welcher nicht durch das exogene Mittel beeinflusst wird und ein oder mehrere dynamische Biomarker zur Identifizierung dynamischer Änderungen in der Wegeaktivität, welche sich aus dem exogenen Mittel ergibt, enthält.

5. Verfahren nach Anspruch 1, wobei das Biomarker-Responder-Paket einen oder mehrere Vorhersage-Biomarker zum Identifizieren eines deregulierten Weges enthält.

6. Verfahren nach Anspruch 1, wobei der Test einen oder mehrere Signalwege erfasst, welche durch Arzneimittelbehandlung heruntergeregelt sind.

7. Verfahren nach Anspruch 6, wobei, basierend auf dem Test, Patienten aus der Responder-Patientenpopulation ausgeschlossen sind, aufgrund der Unfähigkeit des Arzneimittels, den Krankheitsweg zu modulieren.

8. Verfahren nach Anspruch 1, wobei der Test einen oder mehrere erkrankungsrelevante Signalwege erfasst, welche hoch reguliert sind.

9. Verfahren nach Anspruch 8, wobei, basierend auf dem Test, Patienten in die Responder-Patientenpopulation eingeschlossen werden.

10. Verfahren nach Anspruch 1, wobei die Biomarker solche Biomarker sind, die den Aktivitätszustand eines Weges wiedergeben.

11. Verfahren nach Anspruch 1, wobei die Marker Antikörper sind, welche gegen Phosphoproteine gerichtet sind.

12. Ein in vitro Verfahren zum Identifizieren eines Säugers, welcher auf ein Arzneimittel zum Behandeln einer Erkrankung reagiert, umfassend:
(a) Etablieren eines zellulären Modells eines Erkankungsraumes für die Krankheit, basierend auf einem oder mehreren Signalwegen;
(b) Bestimmen eines Biomarker-Responder-Paketes, mit einer Mehrzahl von Biomarkern, wobei die Biomarker spezifisch für einen oder mehrere Signalwege sind und die Biomarker Antikörper sind, die gegen posttranslational modifizierte Proteine gerichtet sind;
(c) Nehmen eines Gewebes, welches von dem Säuger erhalten wurde;
(d) Testens des Gewebes mit dem Biomarker-Responder-Paket; und
(e) Identifizieren, ob der Säuger auf das Arzneimittel, basierend auf dem Test, reagiert.

13. Ein in vitro Verfahren zum Identifizieren einer Säugerpopulation, welche auf ein Arzneimittel zur Behandlung einer Erkrankung reagiert, umfassend:
(a) Etablieren eines zellulären Modells des Krankheitsraumes für die Erkrankung, basierend auf einem oder mehreren Signalwegen;
(b) Bestimmen eines Biomarker-Responder-Pakets mit einer Mehrzahl von Biomarkern, wobei die Biomarker spezifisch sind für einen oder mehrere der Signalwege und die Biomarker Antikörper sind, welche gegen posttranslationale modifizierte Proteine gerichtet sind;
(c) Identifizieren einer Zelllinie, welche für die Säugerpopulation repräsentativ ist;
(d) Testen des Arzneimittels in vitro in der Zelllinie mit dem Biomarker-Responder-Paket; und
(e) Identifizieren, ob die Säugerpopulation auf das Arzneimittel, basierend auf dem Test, reagiert.

## Revendications

1. Procédé *in vitro* permettant d'identifier une population de patients répondeurs pour un traitement avec un agent exogène comprenant :
(a) l'établissement d'un modèle cellulaire d'espace pathologique en se basant sur une ou plusieurs voies de signalisation ;
(b) l'identification de l'effet de l'agent exogène dans la une ou plusieurs voies de signalisation ;
(c) la détermination d'un ensemble biomarqueurs répondeurs comprenant une pluralité de biomarqueurs, où la pluralité des biomarqueurs est spécifique d'une ou de plusieurs des voies de signalisation, et les biomarqueurs sont des anticorps dirigés contre des protéines modifiées après la traduction ;
(d) l'analyse de l'état de la une ou plusieurs voies de signalisation avec l'ensemble biomarqueurs répondeurs avant et après l'administration du médicament ; et
(e) l'identification de la population de patients répondeurs qui seront sensibles à l'agent exogène en se basant sur l'analyse.

2. Procédé selon la revendication 1, dans lequel une ou plusieurs des voies de signalisation sont perturbées ou actives.

3. Procédé selon la revendication 1, dans lequel l'ensemble biomarqueurs répondeurs contient entre : une limite inférieure de trois ; et une limite supérieure de vingt.

4. Procédé selon la revendication 1, dans lequel l'ensemble biomarqueurs répondeurs contient un ou plusieurs biomarqueurs d'exclusion pour identifier une voie qui n'est pas affectée par l'agent exogène et un ou plusieurs biomarqueurs dynamiques pour identifier des changements dynamiques dans l'activité de la voie résultant de l'agent exogène.

5. Procédé selon la revendication 1, dans lequel l'ensemble biomarqueurs répondeurs contient un ou plusieurs biomarqueurs prédictifs pour identifier une voix dérégulée.

6. Procédé selon la revendication 1, dans lequel l'analyse détecte une ou plusieurs voies de signalisation qui sont régulées à la baisse par traitement médicamenteux.

7. Procédé selon la revendication 6, dans lequel en se basant sur l'analyse, des patients sont exclus de la population des patients répondeurs en se basant sur l'incapacité du médicament à moduler lesdites voies pathogènes.

8. Procédé selon la revendication 1, dans lequel l'analyse détecte une ou plusieurs voies de signalisation pertinentes pour la maladie qui sont régulées à la hausse.

9. Procédé selon la revendication 8, dans lequel en se basant sur l'analyse, des patients sont inclus dans la population des patients répondeurs.

10. Procédé selon la revendication 1, dans lequel les biomarqueurs sont des biomarqueurs de l'état de l'activité des voies.

11. Procédé selon la revendication 1, dans lequel les biomarqueurs sont des anticorps dirigés contre des phosphoprotéines.

12. Procédé *in vitro* permettant d'identifier un mammifère sensible à un médicament pour traiter une maladie comprenant :
(a) l'établissement d'un modèle cellulaire d'espace pathogène pour la maladie en se basant sur une ou plusieurs voies de signalisation ;
(b) la détermination d'un ensemble biomarqueurs répondeurs comprenant une pluralité de biomarqueurs, où les biomarqueurs sont spécifiques d'une ou de plusieurs des voies de signalisation et les biomarqueurs sont des anticorps dirigés contre des protéines modifiées après la traduction ;
(c) le prélèvement d'un tissu obtenu d'un mammifère ;
(d) l'analyse du tissu avec l'ensemble biomarqueurs répondeurs ; et
(e) l'identification si le mammifère est sensible au médicament se basant sur l'analyse.

13. Procédé *in vitro* permettant d'identifier une population de mammifères sensibles à un médicament pour traiter une maladie comprenant :
(a) l'établissement d'un modèle cellulaire d'espace pathogène pour la maladie en se basant sur une ou plusieurs voies de signalisation ;
(b) la détermination d'un ensemble biomarqueurs répondeurs comprenant une pluralité de biomarqueurs, où les biomarqueurs sont spécifiques d'une ou de plusieurs des voies de signalisation, et les biomarqueurs sont des anticorps dirigés contre des protéines modifiées après la traduction ;
(c) l'identification d'une lignée cellulaire représentative de la population de mammifères ;
(d) l'analyse du médicament *in vitro* dans la lignée cellulaire avec l'ensemble biomarqueurs répondeurs ; et
(d) l'identification si la population de mammifères est sensible au médicament en se basant sur l'analyse.
